# EUROPÄISCHE PATENTANMELDUNG

(11) **EP 1 526 126 A1**
(43) Veröffentlichungstag der Anmeldung: **27.04.2005**
(21) Anmeldenummer: 04025212.4
(22) Anmeldetag: 22.10.2004
(51) Int. Cl.: C07C 67/333, C07C 69/587, C11C 3/14

(54) **Verfahren zur Herstellung konjugierter, mehrfach ungesättigter Fettsäureester**

(30) Priorität: 23.10.2003 DE 10349455
(71) Anmelder: Bioghurt Biogarde GmbH & Co. KG., 85354 Freising (DE)
(72) Erfinder: Wenk, Hans Henning, 85356 Freising (DE); Häser, Katrin, 82178 Puchheim (DE)
(74) Vertreter: Dey, Michael, Dipl.-Chem. Dr.

(57) **Zusammenfassung**

Das beanspruchte Verfahren zur Herstellung konjugierter mehrfach ungesättigter Fettsäureester, bestehend aus einem Alkoholrest R₁ mit 1 bis 5 Kohlenstoffatomen und einem Fettsäurerest R₂ mit 10 bis 24 Kohlenstoffatomen, mit Alkoholaten mit 1 bis 5 Kohlenstoffatomen ist dadurch gekennzeichnet, dass die Reaktion lösemittelfrei mit Hilfe eines Phasentransferkatalysators durchgeführt wird. Bei diesem Verfahren sollten die Anteile des Fettsäureesters 85 bis 99,9 Gew.-%, die der Alkoholatkomponente 0,05 bis 10 Gew.-% und die des Phasentransferkatalysators 0,05 bis 5,0 Gew.-%, jeweils bezogen auf das Gesamt-Reaktionsgemisch, betragen. Ester von Linolsäure und Linolensäure werden als Ausgangsmaterial ebenso wie Natrium- oder Kaliummethylat bzw. Natrium- oder Kaliumethylat als Alkoholat bevorzugt. Geeignete Phasentransferkatalysatoren sind zum Beispiel Polyethylenglykole, Polyethylenglykolmono- oder -Dimethylether, Kronen-Ether und quartäre Ammoniumoder Phosphoniumsalze. Mit diesem Verfahren, das bevorzugt in einem zweiphasigen System bei Drucken zwischen 1,0 und 2,0 bar und Verfahrenstemperaturen von 60 bis 150° C durchgeführt wird, können Produkte erhalten werden, bei denen mindestens 90 % der theoretisch isomerisierbaren Doppelbindungen in konjugierter Form vorliegen. Das beanspruchte Verfahren wird insbesondere zur Gewinnung konjugierter Linolsäuremethyl- oder -Ethylester herangezogen und zeichnet sich insbesondere durch seine äußerst einfache und wirtschaftliche Verfahrensweise aus.

## Beschreibung

Gegenstand der vorliegenden Erfindung ist ein Verfahren zur Herstellung konjugierter mehrfach ungesättigter Fettsäureester.

Innerhalb der natürlich vorkommenden Gruppe der mehrfach ungesättigten Fettsäuren unterscheidet man in Abhängigkeit von der Stellung der Doppelbindungen zueinander zwischen Fettsäuren mit isolierten Doppelbindungen und Fettsäuren mit konjugierten Doppelbindungen. Während an den erstgenannten Fettsäuren ein natürliches Überangebot besteht, kommen konjugierte Fettsäuren in Naturfetten verhältnismäßig selten vor.

Da gerade die konjugierten Fettsäuren aus technologischer Sicht, aber auch insbesondere unter ernährungsphysiologischen Aspekten in den letzten Jahren immer mehr an Bedeutung gewonnen haben, hat es nicht an Versuchen gefehlt, Fettsäuren mit isolierten Doppelbindungen in Fettsäuren mit konjugierten Doppelbindungen mit Hilfe geeigneter Isomerisierungsreaktionen umzuwandeln.

Ausgehend von der Beobachtung, dass bei der Hydrierung von Fetten konjugiert ungesättigte Derivate entstehen, hat man ursprünglich versucht, die Isomerisierung mit Katalysatoren durchzuführen, wie sie aus Fetthärtungsanwendungen bekannt waren. Zwar konnten die dafür verwendeten wirksamen Katalysatoren in kleinen Mengen eingesetzt werden, die damit erzielte Isomerisierung verlief jedoch nicht quantitativ, wobei außerdem unerwünschte Nebenprodukte, wie polymerisierte oder intramolekular zyklisierte Nebenprodukte auftraten.

Die Isomerisierung von isolierte Doppelbindungen enthaltenden Fettsäuren mit Hilfe basischer Stoffe ist zwar seit langem vorrangig zur quantitativen Bestimmung von mehrfach ungesättigten Fettsäuren, wie zum Beispiel der Linolsäure und der Linolensäure, bekannt. Diese Verfahrensvariante hat sich aber auch geeignet gezeigt, um konjugierte Fettsäuren im Rahmen einer Isomerisierung zu gewinnen. Üblicherweise wirken dabei Alkalihydroxyde oder Alkalialkoholate auf die Fettsäuren oder deren Derivate in mindestens equimolaren Mengen in alkoholischen Lösemitteln bei erhöhten Temperaturen ein.

Gegenstand der vorliegenden Erfindung ist ein Verfahren zur Herstellung konjugierter mehrfach ungesättigter Fettsäureester.

Innerhalb der natürlich vorkommenden Gruppe der mehrfach ungesättigten Fettsäuren unterscheidet man in Abhängigkeit von der Stellung der Doppelbindungen zueinander zwischen Fettsäuren mit isolierten Doppelbindungen und Fettsäuren mit konjugierten Doppelbindungen. Während an den erstgenannten Fettsäuren ein natürliches Überangebot besteht, kommen konjugierte Fettsäuren in Naturfetten verhältnismäßig selten vor.

Da gerade die konjugierten Fettsäuren aus technologischer Sicht, aber auch insbesondere unter ernährungsphysiologischen Aspekten in den letzten Jahren immer mehr an Bedeutung gewonnen haben, hat es nicht an Versuchen gefehlt, Fettsäuren mit isolierten Doppelbindungen in Fettsäuren mit konjugierten Doppelbindungen mit Hilfe geeigneter Isomerisierungsreaktionen umzuwandeln.

Ausgehend von der Beobachtung, dass bei der Hydrierung von Fetten konjugiert ungesättigte Derivate entstehen, hat man ursprünglich versucht, die Isomerisierung mit Katalysatoren durchzuführen, wie sie aus Fetthärtungsanwendungen bekannt waren. Zwar konnten die dafür verwendeten wirksamen Katalysatoren in kleinen Mengen eingesetzt werden, die damit erzielte Isomerisierung verlief jedoch nicht quantitativ, wobei außerdem unerwünschte Nebenprodukte, wie polymerisierte oder intramolekular zyklisierte Nebenprodukte auftraten.

Die Isomerisierung von isolierte Doppelbindungen enthaltenden Fettsäuren mit Hilfe basischer Stoffe ist zwar seit langem vorrangig zur quantitativen Bestimmung von mehrfach ungesättigten Fettsäuren, wie zum Beispiel der Linolsäure und der Linolensäure, bekannt. Diese Verfahrensvariante hat sich aber auch geeignet gezeigt, um konjugierte Fettsäuren im Rahmen einer Isomerisierung zu gewinnen. Üblicherweise wirken dabei Alkalihydroxyde oder Alkalialkoholate auf die Fettsäuren oder deren Derivate in mindestens equimolaren Mengen in alkoholischen Lösemitteln bei erhöhten Temperaturen ein.

Auch in diesem Falle hat es sich aber als nachteilig herausgestellt, dass Nebenreaktionen mehr oder weniger stark ausgeprägt stattfinden, wobei hauptsächlich Polymerisierungs- und Zyklisierungsreaktionen zu beobachten sind; die theoretisch erwarteten Ausbeuten an konjugierten Fettsäuren sind so in der Praxis nicht zu erreichen.

Zur Überwindung dieser Nachteile wurde versucht, die Isomerisierungsbedingungen und hier insbesondere die Konzentration der Alkaliverbindungen, die Temperatur und die Reaktionsdauer so zu verändern, dass die Nebenreaktionen zurückgedrängt werden. Dabei hat sich herausgestellt, dass insbesondere die Art der Alkaliverbindungen und des gewählten Alkohols maßgeblich zum Isomerisierungserfolg beitragen.

In den letzten Jahren haben sich insbesondere drei Verfahrensvarianten durchgesetzt, bei denen die Herstellung konjugierter Fettsäuren und insbesondere die Herstellung einer konjugierten Linolsäure durch Basenkatalyse erfolgt.

Beim ersten Verfahren werden unkonjugierte Fettsäuren oder deren Ester mit Alkalihydroxiden oder anderen Basen in einem hochsiedenden Alkohol, wie zum Beispiel Ethylenglykol (US 2,242,230) in Propylenglykol (z. B. EP-A 0 838 997, US 5,986,116, EP-A 902 082), in Glycerin (vergl. WO 2001/18161) in Polyetheralkohol (z.B. WO 2001/51597) und in Polyolen mit einer freien OH-Gruppe (US 2,343,644) üblicherweise bei Normaldruck umgesetzt. Möglich ist auch die Reaktion in Wasser (z.B. WO 2001/40419, US 4,164,501, US 2,350,583 und GB 561 803) unter dann gleichzeitig hohem Druck, wobei die Reaktionstemperaturen bei den Verfahrensvarianten mit Wasser als Lösemittel über 180° C liegen. In all diesen Fällen wird eine überstöchiometrische Menge einer Basenkomponente zugegeben, da die Base zunächst mit der Fettsäure oder deren Ester zum Salz der Fettsäure im Rahmen einer Verseifungsreaktion reagiert. Außerdem ist zwingend ein Lösemittel erforderlich, um das Reaktionsgemisch flüssig zu halten.

Üblicherweise werden hierfür niedrigsiedende Lösemittel, wie Wasser verwendet, wobei dann allerdings die Gesamtreaktion unter hohen Drucken durchgeführt werden muss. Mit Wasser als Lösemittel läuft die Reaktion zudem erst bei sehr hohen Temperaturen mit einer ausreichenden Geschwindigkeit ab, wobei ein relativ hoher Anteil unerwünschter Fettsäureisomeren entsteht.

Das zweite bekannte Verfahren zur Isomerisierung ungesättigter Fettsäuren bedient sich ausgewählter Fettsäurealkylester als Ausgangsmaterial und verwendet die entsprechenden Alkoholate, also zum Beispiel Fettsäuremethylester und Alkalimethanolat als Isomerisierungskatalysatoren. Da bei diesem Verfahren generell das Problem der Verseifung nicht auftritt, genügen zumeist geringe Mengen an Katalysator von ca. 1 bis 2 Gew.-%. Nachteilig hierbei ist allerdings, dass sich die Alkoholate in den Fettsäureestern nicht lösen, weshalb zwingend Lösemittel für sowohl die Ester als auch die Alkoholat-Komponente eingesetzt werden müssen.

Zur Durchführung dieser Isomerisierungsvariante sind bereits wenige Prozent des jeweiligen Lösemittels ausreichend, wofür insbesondere Alkohole in Frage kommen, wie sie bereits in Form der Ester und Alkoholate in der Reaktionsmischung vorliegen. Allerdings muss die Reaktion unter erhöhtem Druck durchgeführt werden, da die Reaktionstemperatur für die kurzkettigen Alkohole deutlich über deren jeweiliger Siedetemperatur liegt. Zudem müssen entsprechende technische Maßnahmen ergriffen werden.

Entsprechende Reaktionen von unkonjugierten Fettsäurealkylestern mit Alkalialkoholaten in Gegenwart der entsprechenden Alkohole als Lösemittel und bei Temperaturen über 100 °C unter Druckbedingungen sind beispielsweise aus US 6,479,683, DE-AS 1 156 788 sowie DE-AS 1 156 789 bekannt.

Als dritte Verfahrensvariante zur Isomerisierung unkonjugierter Fettsäurederivate hat sich eine Reaktionsweise durchgesetzt, bei der die unkonjugierten Fettsäurealkylester mit Alkalialkoholaten in polar-aprotischen Lösemitteln durchgeführt wird.

Entsprechende Verfahren sind aus DE-OS 2 250 232, DE-OS 2 155 727 und aus US 3,984,444 bekannt.

Diese Reaktionen in polar-aprotischen Lösemitteln laufen zwar bei relativ niedrigen Temperaturen ab, allerdings sind die eingesetzten Substanzen zum Teil toxikologisch bedenklich und zudem schwer aus dem Produkt abzutrennen, was diese Reaktionsvariante im Hinblick auf den Einsatz der Produkte in Lebensmitteln ungeeignet macht.

Wie bereits dargelegt, stellen konjugierte Linolsäuren, Linolensäuren und deren Derivate in Form von Estern wichtige Vertreter aus der Gruppe der Fettsäuren (-derivate) dar.

Bei der konjugierten Linolsäure (CLA) handelt es sich um eine Mischung von positions- und konfigurationsabhängigen Isomeren der Octadecadiensäure, die natürlicherweise in Milch und in Fleisch von Wiederkäuern vorkommt.

Die Abkürzung "CLA" umfasst somit im Wesentlichen C18:2 Fettsäuren und insbesondere die 9-cis-11-trans- und 10-trans-12-cis-Octadecadiensäure.

Neben seiner nachgewiesenen positiven Wirkung bei der Karzinogenese in Brust-, Darm-, Magen- und Hautgeweben, wobei es insbesondere modulierend auf Lymphozyten- und Makrophagen-Aktivitäten wirkt, stellt CLA auch einen biologisch aktiven Inhaltsstoff für Nahrungsergänzungsmittel, insbesondere in Verbindung mit Antioxidantien dar.

Insgesamt ist CLA somit ein prominenter Vertreter der konjugierten Fettsäuren und deren Derivate.

Ein Beispiel für die Herstellung von CLA stellt das gemäß WO 2001/51597 beschriebene Verfahren dar. Hierbei wird ein mit Fettsäuren angereichertes Öl mit katalytischen Mengen einer Basenkomponente in einem Medium durchgeführt, das einen Polyetheralkohol (zum Beispiel Polyethylenglykol) als Lösemittel enthält. Die Reaktionstemperatur sollte dabei über 90° C liegen. Auch hier ist zunächst eine stöchiometrische Menge der Basenkomponente erforderlich, um das Öl oder die Fettsäuren in die Seifen-Form zu überführen.

Gemäß US 2,343,644 erfolgt die Konjugation eines fettartigen Polyens mit einem Überschuss einer Basenkomponente und in Gegenwart eines Ethers eines mehrbasigen Alkohols, der eine freie OH-Gruppe enthält. In Frage kommen hierfür insbesondere Polyethylenglykolmonomethylether.

Nachteilig hierbei ist das zwingend erforderliche Arbeiten mit einem Überschuss an Base und zudem ist dieses Verfahren auf Ether mehrbasiger Alkohole beschränkt, die eine freie OH-Gruppe aufweisen.

Für das vorliegende Verfahren hat sich deshalb die Aufgabe gestellt, ein Verfahren zur Herstellung konjugierter mehrfach ungesättigter Fettsäureester bereitzustellen, bei dem die Umsetzung nicht konjugierter mehrfach ungesättigter Fettsäureester, bestehend aus einem Alkoholrest R₁
mit 1 bis 5 Kohlenstoffatomen und einem Fettsäurerest R₂ mit 10 bis 24 Kohlenstoffatomen, mit C₁₋₅-Alkoholaten im Mittelpunkt steht. Dieses Verfahren soll möglichst einfach durchzuführen sein; d.h. es soll keiner Temperatur- und Druckbeschränkung unterliegen und die eingesetzten Ausgangsmaterialien sowie das Reaktionsmedium selbst sollen auf wenige Komponenten beschränkt sein. Dabei sollen Nebenreaktionen mit den entsprechenden unerwünschten Produkten vermieden werden und die mit dem neuen Verfahren erhaltenen konjugierten, mehrfach ungesättigten Fettsäureester sollen in ausreichenden Ausbeuten und guten Reinheiten zugänglich sein.

Gelöst wurde diese Aufgabe mit einem entsprechenden Verfahren, bei dem die Reaktion lösemittelfrei mit Hilfe eines Phasentransferkatalysators durchgeführt wird.

Überraschend hat sich bei der Umsetzung dieses Verfahrens herausgestellt, dass der Isomerisierungsgrad der erhaltenen Produkte regelmäßig über 90 % liegt, wobei die Reaktionsbedingungen im Vergleich zu den aus dem Stand der Technik bekannten Verfahren relativ einfach gehalten werden können, da die weiteren Reaktionsbedingungen wie Temperatur und Druck keinerlei spezielle Anforderungen an die Versuchsdurchführung und die Reaktionsvorrichtung stellen. Es war nicht zu erwarten, dass mit dieser einfachen Reaktionsführung Produktqualitäten erreicht werden, die über den bekannten Qualitäten vergleichbarer Verfahren liegen.

Im Hinblick auf den eingesetzten Fettsäureester haben sich Einsatzmengen von 85 bis 99,9 Gew.-% und bezüglich des eingesetzten Alkoholats solche von 0,05 bis 10 Gew.-%, jeweils bezogen auf die Gesamtreaktionsmischung, als besonders geeignet erwiesen. Dabei werden Mengen des Fettsäureesters zwischen 90,0 und 98,0 Gew.-% und des Alkoholats zwischen 0,5 und 5,0 Gew.-%, wieder jeweils bezogen auf die Gesamtreaktionsmischung, als besonders bevorzugt angesehen.

Zwar kann das beanspruchte Verfahren innerhalb der genannten Grenzen mit einer Vielzahl von Fettsäureestern durchgeführt werden, doch wird eine Verfahrensvariante bevorzugt, bei der Ester von Linolsäure und Linolensäure als Ausgangsmaterial verwendet werden.

Aus der Gruppe der Alkoholate mit 1 bis 5 Kohlenstoffatomen haben sich Natrium- und Kaliumalkoholate und insbesondere das Natrium- und Kaliummethylat aber auch Natrium- und Kaliumethylat als besonders geeignet gezeigt.

Im Gegensatz zu den bislang bekannten Verfahren, die entweder unter Zuhilfenahme von organischen Lösemitteln arbeiten oder aber in Gegenwart von Wasser, wird das erfindungsgemäße Verfahren lösemittelfrei durchgeführt, wobei als weiteres erfindungswesentliches Merkmal die Verwendung eines Phasentransferkatalysators im Vordergrund steht. Dieser Phasentransferkatalysator erhöht die Löslichkeit des Alkoholates in den Fettsäureestern und ermöglicht dadurch die Isomerisierung. Gleichzeitig wird im Gegensatz zu den niedrigsiedenden Alkoholen als Lösemittel das Arbeiten unter hohem Druck vermieden, da der Dampfdruck der Phasentransferkatalysatoren äußerst gering ist.

Die Menge an eingesetztem Phasentransferkatalysator ist lediglich wirtschaftlichen Limitierungen unterworfen, wobei sich allerdings Mengen von 0,05 bis 5,0 Gew.-% und besonders bevorzugt Mengen von 0,5 bis 1,5 Gew.-%, jeweils bezogen auf das Gesamtreaktionsgemisch, als besonders geeignet gezeigt haben. Als bevorzugte Vertreter des Phasentransferkatalysators kommen Polyethylenglykole, Polyethylenglykolmono- oder dimethylether, Kronen-Ether, wie z. B. 15-Krone-5, 18-Krone-6, Dibenzo-18-krone-6, Dicyclohexano-18-krone-6, quartäre Ammonium- oder Phosphoniumsalze, wie z.B. Benzyltrimethylammoniumchlorid, Cetyltrimethylammoniumbromid, Tetrabutylammoniumbromid, Benzyltriphenylphosphoniumbromid oder Tetrabutylphosphoniumbromid und deren Mischungen in Frage, wobei nicht toxische Phasentransferkatalysatoren wie z.B. Polyethylenglykole insbesondere geeignet sind.

Hinsichtlich des Reaktionsmediums bevorzugte vorliegende Erfindung eine Variante, bei der die Konjugation in einem zweiphasigen System durchgeführt wird.

Wie bereits mehrfach angedeutet, ist der besondere Vorteil des vorliegenden Verfahrens darin zu sehen, dass keine speziellen Bedingungen hinsichtlich des Reaktionsdruckes und der Reaktionstemperatur eingehalten werden müssen. Vor allem aus wirtschaftlichen Gründen ist allerdings eine Reaktionsführung bevorzugt, die bei Drucken zwischen 1,0 und 2,0 bar und besonders bevorzugt bei Normaldruck durchgeführt wird. Hinsichtlich der zu wählenden Verfahrenstemperaturen sind Bereiche zwischen 60 und 150 °C, besonders bevorzugt zwischen 80 und 120 °C und ganz besonders bevorzugt zwischen 90 und 100 °C als geeignet anzusehen.

Selbstverständlich nimmt die Reaktionszeit mit abnehmender Reaktionstemperatur immer mehr zu. Jedoch hat sich als zusätzlich überraschend herausgestellt, dass der Anteil unerwünschter Isomere im Produkt dann äußerst gering ist, wenn die Reaktionstemperatur möglichst niedrig, d.h., im Bereich um 60 °C gewählt wird. Über die Auswahl der Reaktionstemperatur kann somit im Bedarfsfall der Isomerisierungsgrad bzw. das Verhältnis der unterschiedlichen Isomeren vorgewählt und gesteuert werden. Auch bei höheren Temperaturen (z.B. 100 °C) sind beim erfindungsgemäßen Verfahren unerwünschte Isomere, d.h. insbesondere andere als 9-cis-11-trans- und 10-trans-12-cis-Octadecadiensäure, nicht nachweisbar, wobei natürlich grundsätzlich auch bei den unerwünschten Isomeren die Doppelbindungen in Konjugation zueinander vorliegen können.

Nicht zuletzt aus diesem Grund beansprucht die vorliegende Erfindung auch eine Verfahrensvariante, mit der Produkte erhalten werden, bei denen mindestens 90 % und isnbesondere mindestens 95 % der theoretisch isomerisierbaren Doppelbindungen in konjugierter Form vorliegen. In diesem Zusammenhang ist anzumerken, dass der Isomerisierungsgrad bspw. für die Linolsäure im allgemeinen eindeutig zu bestimmen ist, da z.B. bei vollständigem Umsatz 100% der Doppelbindungen konjugiert sind. Bei Linolensäure und anderen Fettsäuren mit mindestens drei Doppelbindungen ist die Definition schwieriger, da in diesen Fällen Isomere entstehen können, die zwar nicht mehr weiter reagieren können, bei denen aber nicht alle Doppelbindungen konjugiert sind.

Mit umfasst von der vorliegenden Erfindung wird auch ein Abtrennungsschritt für das Produkt, der dem eigentlichen Verfahren nachgeschaltet wird. Dieser zusätzliche Abtrennungsschritt beinhaltet mindestens das Waschen des Produktes mit einer verdünnten Säure, die bevorzugt eine Phosphorsäure ist, und das abschließende Trocknen, vorzugsweise in Vakuum und/oder bei erhöhten Temperaturen.

Aufgrund der bereits geschilderten ernährungsphysiologisch bedeutenden Eigenschaften der konjugierten Linolsäure (CLA) umfasst die vorliegende Erfindung auch ein Verfahren, bei der konjugierte Linolsäuremethylester oder konjugierte Linolsäureethylester erhalten werden.

Zusammenfassend weist das vorgeschlagene Verfahren als Vorteile seine Lösemittelfreiheit und den Einsatz von Phasentransferkatalysatoren auf, die, in nur sehr geringen Mengen eingesetzt, das Arbeiten unter hohen Drücken vermeiden, wobei es auch möglich ist, durch Auswahl des jeweiligen Temperaturbereichs den Anteil der unterschiedlichen Isomere weitgehend gezielt zu steuern. Aufgrund seiner einfachen Durchführbarkeit und der dreikomponentigen Zusammensetzung, basierend auf dem Fettsäureester, den Alkoholaten und dem Phasentransferkatalysator, ist das vorgeschlagene Verfahren äußerst wirtschaftlich durchzuführen, wobei Produkte hoher Qualität, insbesondere in Form von CLA-Estern erhalten werden.

Das beanspruchte Verfahren zur Herstellung konjugierter mehrfach ungesättigter Fettsäureester, bestehend aus einem Alkoholrest R₁ mit 1 bis 5 Kohlenstoffatomen und einem Fettsäurerest R₂ mit 10 bis 24 Kohlenstoffatomen, mit Alkoholaten mit 1 bis 5 Kohlenstoffatomen ist dadurch gekennzeichnet, dass die Reaktion lösemittelfrei mit Hilfe eines Phasentransferkatalysators durchgeführt wird. Bei diesem Verfahren sollten die Anteile des Fettsäureesters 85 bis 99,9 Gew.-%, die der Alkoholatkomponente 0,05 bis 10 Gew.-% und die des Phasentransferkatalysators 0,05 bis 5,0 Gew.-%, jeweils bezogen auf das Gesamt-Reaktionsgemisch, betragen. Ester von Linolsäure und Linolensäure werden als Ausgangsmaterial ebenso wie Natrium- oder Kaliummethylat bzw. Natrium- oder Kaliumethylat als Alkoholat bevorzugt. Geeignete Phasentransferkatalysatoren sind zum Beispiel Polyethylenglykole, Polyethylenglykolmono- oder -Dimethylether, Kronen-Ether und quartäre Ammonium- oder Phosphoniumsalze. Mit diesem Verfahren, das bevorzugt in einem zweiphasigen System bei Drucken zwischen 1,0 und 2,0 bar und Verfahrenstemperaturen von 60 bis 150° C durchgeführt wird, können Produkte erhalten werden, bei denen mindestens 90 % der theoretisch isomerisierbaren Doppelbindungen in konjugierter Form vorliegen. Das beanspruchte Verfahren wird insbesondere zur Gewinnung konjugierter Linolsäuremethyl- oder -Ethylester herangezogen und zeichnet sich insbesondere durch äußerst einfache und wirtschaftliche Verfahrensweise aus.

Die nachfolgenden Beispiele belegen die Vorteile des beanspruchten Verfahrens zur Herstellung konjugierter mehrfach ungesättigter Fettsäureester.

### Beispiele

### Beispiel 1 (Vergleichsbeispiel)

### Isomerisierung ohne Phasentransferkatalysator

50 g Fettsäuremethylester (76 % Linolsäure im Fettsäureanteil) wurden mit 1 g Kaliummethylat drei Stunden lang bei 100 °C gerührt. Die Reaktion wurde durch Zugabe von 1,0 ml einer 85 %igen Phosphorsäure beendet und das Produkt zweimal mit Wasser gewaschen. Es wurde eine orange Flüssigkeit mit einem Isomerisierungsgrad von 6 % (Bestimmung mittels Gaschromatographie) erhalten.

### Beispiel 2 (Erfindung)

### Isomerisierung mit einem Phasentransferkatalysator

20 g Fettsäureethylester (76 % Linolsäure im Fettsäureanteil) wurden mit 0,2 g Polyethylenglykol (Phasentransferkatalysator mit einer mittleren Molmasse 400) und 0,4 g Kaliumethylat (Alkoholatkomponente) 210 Minuten lang unter Rühren und unter einer Stickstoffatmosphäre auf 100 °C erhitzt. Anschließend wurde die Reaktion durch Zugabe von 1,0 ml einer 85 %-igen Phosphorsäure beendet und das Produkt wurde zweimal mit Wasser gewaschen. Es wurde eine orange Flüssigkeit mit einem Isomerisierungsgrad von 93 % (Bestimmung mittels Gaschromatographie) erhalten.

Der direkte Vergleich des Isomerisierungsgrades der Produkte aus Beispiel 1 und Beispiel 2 zeigt den positiven Einfluss des Phasentransferkatalysators bei sonst annähernd identischer Reaktionsführung.

### Beispiel 3 (Erfindung)

380 g Fettsäuremethylester (76 % Linolsäure im Fettsäureanteil) und hergestellt durch Umesterung von Färberdistelöl mit Methanol in Gegenwart von 1 % Kaliumhydroxyd) wurden mit 3,82 g Polyethylenglykol (mittlere Molmasse 400) und 5,74 g Kaliummethylat 210 Minuten lang unter kräftigem Rühren auf 95 °C erhitzt. Die Reaktion wurde insgesamt unter einer Stickstoffatmosphäre durchgeführt. Abschließend wurde das Reaktionsgemisch dreimal mit je 100 ml einer 2 %-igen Phosphorsäure gewaschen und das Produkt im Vakuum bei 80° C getrocknet. Man erhielt 370 g einer schwach gelben Flüssigkeit mit einem Isomerisierungsgrad > 99 %. Der Gehalt an konjugierter Linolsäure im Fettsäureanteil wurde mit Hilfe der Gaschromatographie als 76 % bestimmt, wovon je die Hälfte auf die 9-cis-11-trans- und 10-trans-12-cis-Octadecadiensäure entfielen.

## Patentansprüche

1. Verfahren zur Herstellung konjugierter, mehrfach ungesättigter Fettsäureester durch Umsetzung nicht konjugierter, mehrfach ungesättigter Fettsäureester bestehend aus einem Alkoholrest R₁ mit 1 bis 5 Kohlenstoffatomen und einem Fettsäurerest R₂ mit 10 bis 24 Kohlenstoffatomen, mit Alkoholaten mit 1 bis 5 Kohlenstoffatomen, **dadurch gekennzeichnet, dass** die Reaktion lösemittelfrei mit Hilfe eines Phasentransferkatalysators durchgeführt wird.

2. Verfahren nach Anspruch 1, **dadurch gekennzeichnet, dass** 85 bis 99,9 Gew.-% des Fettsäureesters und 0,05 bis 10 Gew.-% des Alkoholats, jeweils bezogen auf die Gesamt-Reaktionsmischung, eingesetzt werden.

3. Verfahren nach einem der Ansprüche 1 oder 2, **dadurch gekennzeichnet, dass** 90,0 bis 98,0 Gew.-% des Fettsäureesters und 0,5 bis 5,0 Gew.-% des Alkoholats, jeweils bezogen auf die Gesamt-Reaktionsmischung, eingesetzt werden.

4. Verfahren nach einem der Ansprüche 1 bis 3, **dadurch gekennzeichnet, dass** Ester von Linolsäure und Linolensäure als Ausgangsmaterial verwendet werden.

5. Verfahren nach einem der Ansprüche 1 bis 4, **dadurch gekennzeichnet, dass** ein Natrium- oder Kaliummethylat oder -Ethylat als Alkoholat eingesetzt wird.

6. Verfahren nach einem der Ansprüche 1 bis 5, **dadurch gekennzeichnet, dass** der Phasentransferkatalysator in Mengen von 0,05 bis 5,0 Gew.-% und besonders bevorzugt in Mengen von 0,5 bis 1,5 Gew.-%, jeweils bezogen auf das Gesamt-Reaktionsgemisch, eingesetzt wird.

7. Verfahren nach einem der Ansprüche 1 bis 6, **dadurch gekennzeichnet, dass** als Phasentransferkatalysator Polyethylenglykole, Polyethylenglykolmono- oder dimethylether, Kronen-Ether, wie z. B. 15-Krone-5, 18-Krone-6, Dibenzo-18-krone-6, Dicyclohexano-18-krone-6, quartäre Ammonium- oder Phosphoniumsalze, wie z. B. Benzyltrimethylammoniumchlorid, Cetyltrimethylammoniumbromid, Tetrabutylammoniumbromid, Benzyltriphenylphosphoniumbromid oder Tetrabutylphosphoniumbromid und deren Mischungen verwendet werden.

8. Verfahren nach einem der Ansprüche 1 bis 7, **dadurch gekennzeichnet, dass** die Konjugation in einem 2-phasigen System durchgeführt wird.

9. Verfahren nach einem der Ansprüche 1 bis 8, **dadurch gekennzeichnet, dass** die Reaktion bei Drucken zwischen 1,0 und 2,0 bar und besonders bevorzugt bei Normaldruck durchgeführt wird.

10. Verfahren nach einem der Ansprüche 1 bis 9, **dadurch gekennzeichnet, dass** die Reaktion bei Verfahrenstemperaturen von 60 bis 150 °C, besonders bevorzugt bei 80 bis 120 °C und ganz besonders bevorzugt zwischen 90 und 100 °C durchgeführt wird.

11. Verfahren nach einem der Ansprüche 1 bis 10, **dadurch gekennzeichnet, dass** Produkte erhalten werden, bei denen mindestens 90 % der theoretisch isomerisierbaren Doppelbindungen in konjugierter Form vorliegen.

12. Verfahren nach einem der Ansprüche 1 bis 11, **dadurch gekennzeichnet, dass** ein Abtrennungsschritt für das Produkt nachgeschaltet wird, der mindestens das Waschen des Produktes mit einer verdünnten Säure, bevorzugt einer Phosphorsäure, und das Trocknen, bevorzugt im Vakuum und/oder bei erhöhten Temperaturen, umfasst.

13. Verfahren nach einem der Ansprüche 1 bis 11, **dadurch gekennzeichnet, dass** konjugierte Linolsäuremethyl- oder -ethyl-Ester erhalten werden.
